**Europäisches Patentamt**

**European Patent Office**

**Office européen des brevets**

(11) Numéro de publication : **0 395 547 B1**

# FASCICULE DE BREVET EUROPEEN

(45) Date de publication du fascicule du brevet :
**28.07.93 Bulletin 93/30**

(51) Int. Cl.$^5$ : **C07C 69/145,** C07C 67/293

(21) Numéro de dépôt : **90420205.8**

(22) Date de dépôt : **24.04.90**

(54) **Procédé de préparation de carboxylates de cyclohexène-2 yle.**

(30) Priorité : **28.04.89 FR 8906020**

(43) Date de publication de la demande :
**31.10.90 Bulletin 90/44**

(45) Mention de la délivrance du brevet :
**28.07.93 Bulletin 93/30**

(84) Etats contractants désignés :
**AT BE CH DE ES FR GB GR IT LI LU NL SE**

(56) Documents cités :
**CHEMISCHE BERICHTE, vol. 141, 1988, pages 1151-1158, VCH, Verlagsgesellschaft mbH, Weinheim, DE; D. HERMELING et al.: "Cyclische Olefine durch anodische Oxidation von beta-(Trimethylsilyl)carbonsäuren. - beta-(Trimethylsilyl)acrylsäure-Derivate als Acetylen-Äquivalente in Diels-Alder-Reaktionen)**

(73) Titulaire : **RHONE-POULENC CHIMIE
25, quai Paul Doumer
F-92408 Courbevoie Cédex (FR)**

(72) Inventeur : **Costantini, Michel
10, Rue du Docteur Bonhomme
F-69003 Lyon (FR)**
Inventeur : **Laucher, Dominique
205, Avenue Jean Jaurès
F-69007 Lyon (FR)**

(74) Mandataire : **Dubruc, Philippe et al
RHONE-POULENC CHIMIE Direction de la
Propriété Industrielle 25, Quai Paul Doumer
F-92408 Courbevoie Cedex (FR)**

EP 0 395 547 B1

## Description

La présente invention a pour objet un procédé de préparation de carboxylates de cyclohexène-2 yle.

Les carboxylates de cyclohexène-2 yle et, notamment, l'acétate de cyclohexène-2 yle sont des produits utiles comme intermédiaires dans diverses synthèses organiques. Par exemple, l'acétate de cyclohexène-2 yle peut être hydrolysé pour former le cyclohexène-2 ol-1. Ce produit peut alors être déshydrogéné de manière en soi connue pour former le phénol, ou déshydraté en cyclohexadiène, intermédiaire utile pour préparer des monomères entrant dans la fabrication de polyesters ou de polyamides.

Il a déjà été proposé de préparer l'acétate de cyclohexène-2 yle par oxydation du cyclohexène en milieu acétique.

Ainsi dans le brevet français n° 1 358 707 il est indiqué que ce composé est obtenu par oxydation du cyclohexène dans l'anhydride acétique par l'oxygène moléculaire en présence d'un catalyseur à base de cobalt et d'un bromure.

Dans le brevet américain n° 3 632 633 il est proposé de préparer ce composé en traitant le cyclohexène par l'acide nitrique en présence d'une faujasite sous forme acide.

Dans le brevet français n° 2 200 226, ce même ester est préparé par réaction du cyclohexène, de l'acide acétique et de l'hydroperoxyde de cyclohexyle en présence de chlorure cuivrique.

Il est également connu d'après Chem. Ber., 121 (6), 1151-8, 1988 que cet ester peut être obtenu par cycloaddition de l'acétoxy-1 butadiène-1,3 sur un composé diénophile substitué par un groupe triméthylsilyle tel $(CH_3)_3$ SiCH = CHCOCl pour former l'acide β-triméthylsilylcarboxylique cyclique insaturé, suivie de l'hydrogénation de ce dernier en son analogue saturé et de la décarboxylation-désilylation de l'acide saturé pour former l'ester recherché.

Si l'acétoxy-1 butadiène-1,3 est une matière première aisément accessible par oxydation à l'air du butadiène en milieu acétique, les diénophiles substitués par un groupe triméthylsilyle sont des dérivés relativement rares et onéreux. En outre le nombre d'étapes requises pour obtenir l'acétate de cyclohexène-2 yle recherché rend illusoire le développement à l'échelle industrielle d'un tel procédé.

Bien que le cyclohexène soit une matière première disponible, il ne l'est qu'en quantité relativement limitée et à un prix relativement élevé.

C'est pourquoi il serait très intéressant de pouvoir disposer d'un procédé de préparation de carboxylate de cyclohexène-2 yle par cycloaddition d'un carboxylate de butadiène-1,3 yle et d'un composé largement accessible et relativement peu onéreux, sans pour autant être amené à prévoir une multiplicité de réactions chimiques pour accéder aux dits carboxylates.

Les recherches conduites par la Demanderesse ont montré qu'il est possible d'atteindre un tel objectif en préparant les carboxylates de cyclohexène-2 yle en un stade, par cycloaddition d'un carboxylate de butadiène-1,3 yle sur l'éthylène.

La présente invention a donc pour objet un procédé de préparation de carboxylates de cyclohexène-2 yle par cycloaddition d'un carboxylate de butadiène-1,3 yle sur l'éthylène.

Par carboxylates on entend dans le cadre de la présente invention les dérivés des acides carboxyliques renfermant au maximum 4 atomes de carbone et, en particulier, les acétates.

L'addition ou condensation est réalisée par mise en contact de l'éthylène et du carboxylate de butadiène-1,3 yle sous pression supérieure à la pression atmosphérique et à une température supérieure ou égale à 100°C.

Pour une bonne mise en oeuvre de l'invention la température choisie est inférieure à 250°C ; en effet, au delà de cette température on observe des dégradations du carboxylate de butadiène-1,3 yle. De préférence, elle est comprise entre 120 et 230°C.

La pression partielle de l'éthylène (mesurée à 25°C) est en général supérieure ou égale à 5 bar ( 500 KPa) ; il n'est pas utile qu'elle dépasse 200 bar (20 000 KPa). Elle est de préférence comprise entre 30 et 70 bar (3000 et 7000 KPa).

Selon un mode de réalisation particulièrement avantageux la condensation est conduite dans un solvant ou diluant organique.

A cette fin on peut utiliser diverses classes de composés organiques et en particulier :

- les hydrocarbures aromatiques chlorés ou non tels le benzène, le toluène, les xylènes et l'orthodichlorobenzène ;
- les hydrocarbures saturés, aliphatiques ou cycloaliphatiques tels le cyclohexane, le pentadécane et l'hexadécane ;
- les esters tels l'acétate de butyle, et l'acétate d'isopropyle ;
- les éthers tel le diglyme ;
- les oléfines dont la double liaison est stériquement suffisamment encombrée pour ne pas réagir en tant

2

que diénophile sur le carboxylate de butadiène-1,3 yle, par exemple, le diméthyl-2,3 butène-2.

La concentration du carboxylate de butadiène-1,3 yle dans le diluant organique peu varier dans de larges limites. Lorsqu'on opère en discontinu et pour une bonne mise en oeuvre du procédé selon l'invention il est préférable qu'elle n'excède pas 0,5 Mol/l et de bons résultats sont obtenus pour une concentration comprise entre 0,01 et 0,3 Mol/l. Lorsqu'on opère en semi-continu ou continu on peut, bien entendu, introduire le carboxylate de butadiène-1,3 yle-1 progressivement et atteindre une concentration en carboxylate de cyclohexène-2 yle-1 de l'ordre de 1 à 3 mol/l.

La présence d'eau est tolérable dans le milieu réactionnel, même en quantité relativement importante. On observe toutefois une co-production de cyclohexène-2 ol, produit valorisable en lui-même et la conversion globale semble être négativement affectée.

Il peut s'avérer avantageux de conduire la condensation en présence d'un inhibiteur radicalaire tel l'hydroquinone ou le tertiobutyl-4 pyrocatéchol.

La quantité d'inhibiteur à mettre en oeuvre n'est pas critique et est généralement de l'ordre de 0,5 à 10 % poids par rapport au carboxylate de butadiène-1,3 yle.

Si la présence de gaz inertes tels l'argon ou l'azote peut avoir un effet favorable sur le bon déroulement de la réaction, il n'en est pas de même de l'oxygène ou de l'air dont il convient d'éviter la présence.

Comme cela a été indiqué précédemment, dans un objet plus particulier, la présente invention vise la préparation de l'acétate de cyclohexène-2 yle par cycloaddition de l'acétoxy-1 butadiène-1,3 et de l'éthylène.

L'acétoxy-1 butadiène-1,3 est le plus souvent disponible sous forme d'un mélange d'isomère trans et cis.

La Demanderesse a constaté dans le cadre de ses travaux ayant abouti à la présente invention que l'isomère trans de l'acétoxy-1 butadiène-1,3 est plus réactif que l'isomère cis et conduit à une meilleure sélectivité en acétate recherché. Bien que le mélange d'isomères dans le rapport typique trans/cis de l'ordre de 1 à 1,5 convienne à la mise en oeuvre du procédé selon l'invention, selon une variante d'exécution avantageuse du présent procédé on utilise un mélange enrichi en isomère trans.

La durée de réaction (ou temps de séjour des réactifs) peut varier dans de larges limites et peut être déterminée aisément en fonction de la conversion souhaitée et des conditions précises choisies pour la mise en oeuvre de la réaction.

Lorsqu'on opère en discontinu elle est habituellement comprise entre 30 mn et 30 h.

En fin de réaction ou du temps imparti à celle-ci, on récupère le carboxylate de cyclohexène-2 yle par tout moyen approprié, en particulier par distillation.

Les exemples ci-après illustrent la présente invention.

EXEMPLE 1 à 6 :

Mode opératoire.

Dans un autoclave en acier inoxydable de 25 cm³ de capacité on charge 10 cm³ de diluant organique et 0,0984 g (0,879 mMol) d'acétoxy-1, butadiène (mélange d'isomères trans/cis = 1,75).

On ferme l'autoclave et on introduit alors 9 bar d'éthylène ($\simeq$ 6,2 mMol) puis 71 bar d'argon. On place alors l'autoclave dans un four puis on le porte pendant 17 heures à la température indiquée (T), l'agitation étant assurée par balancement.

L'autoclave est alors refroidi et dégazé et la masse réactionnelle est analysée par chromatographie en phase vapeur.

La sélectivité en acétate de cyclohexène-2 yle S(AC) est indiquée par rapport à l'acétoxy-1 butadiène-1,3 transformé.

Les résultats ainsi que les conditions particulières figurent au tableau I ci-après.

TABLEAU I

- - - - - - - - -

| Ex n° | Diluant | T°C | Taux de conversion | | S(AC) |
|-------|---------|-----|------|------|-------|
| | | | Cis | Trans | |
| 1 | Toluène | 130 | 11 % | 12 % | 5,9 % |
| 2 | " | 150 | 14,5 % | 19,5 % | 12 % |
| 3 | " | 170 | 13,5 % | 30 % | 34 % |
| 4 | " | 180 | 23,5 % | 48,5 % | 31 % |
| 5 | Cyclohexane | 150 | 17 % | 20,5 % | 13,5 % |
| 6 | " | 170 | 28 % | 38,5 % | 19,5 % |

EXEMPLE 7 :

Dans un autoclave en acier inoxydable de 25 cm3 on introduit 10 cm3 de toluène et 0,115 g (1,04 mMol) d'acétoxy-1 butadiène-1,3 (isomère trans/isomère cis = 1,56) avec en plus, 4,6 % en poids de t-butyl-4 pyrocatechol par rapport à l'acétoxybutadiène. On introduit 70 bar (48 mMol environ) d'éthylène. On chauffe ensuite à 180°C pendant 3 heures sous agitation par balancement.
Les résultats obtenus sont les suivants :
- Taux de conversion de l'isomère cis : 0
- Taux de conversion de l'isomère trans : 47 %
- Sélectivité en acétoxy-2 cyclohexène : 93 %

EXEMPLE 8 :

Dans un autoclave de 25 cm3 en acier inoxydable revêtu intérieurement de téflon®, on place 10 cm3 de toluène, 0,115 g (1,04 mMol) d'acétoxy-1 butadiène-1,3 (rapport trans/cis : 1,56) et du t. butyl-4 pyrocatechol (4,6 % en poids/acétoxybutadiène). On introduit 40 bar d'éthylène et 30 bar d'argon.
On chauffe ensuite à 180°C pendant 6 heures.
Les résultats obtenus sont les suivants :
- taux de conversion de l'isomère cis : 6 %
- taux de conversion de l'isomère trans : 48,5 %
- Sélectivité en acétoxy-2 cyclohexène : 72,5 %

EXEMPLES 9 A 17 :

On dispose de plusieurs autoclaves de 25 cm3 en acier inoxydable, revêtu intérieurement de téflon®.
Dans chaque autoclave on place 10 cm3 de solvant et 1 mMol d'acétoxy-1 butadiène-1,3 (isomère trans/isomère cis : 1,55) renfermant 4,6 % en poids de tertiobutyl-4 pyrocatéchol. Les autoclaves étant fermés, on introduit, dans chaque autoclave 40 bar d'éthylène et 30 bar d'argon.

Ces autoclaves sont placés chacun dans un four puis chauffés pendant 6 heures à 180°C. L'agitation est assurée par balancement.

Après refroidissement, chaque autoclave est dégazé puis chaque masse réactionnelle est analysée par chromatographie en phase vapeur. Les résultats sont donnés dans le tableau II ci-après.

TABLEAU II
----------

| Ex n° | Diluant | Taux de conversion | | S(AC) |
|---|---|---|---|---|
| | | Cis | Trans | |
| 9 | Toluène | 6 % | 48,5 % | 72,5 % |
| 10 | Acétate de butyle | 21,5 % | 35 % | 55,5 % |
| 11 | Cyclohexane | 13 % | 48 % | 53 % |
| 12 | Pentadécane | 34 % | 69,5 % | 38 % |
| 13 | Hexadécane | 36,5 % | 69,5 % | 38,5 % |
| 14 | Diglyme | 21 % | 56 % | 49 % |
| 15 | Acide acétique | 54 | 99,5 % | NEANT |
| 16 | Diglyme-eau (*) | 81 % | 61 % | 9,5 % |
| 17 | Néant | 92,5 % | 93,5 % | 2,5 % |

(*) (50/50 vol)

EXEMPLES 18 A 20 :

On dispose de 3 autoclaves de 25 cm3 en acier inoxydable revêtu intérieurement de téflon®.

Dans chaque autoclave on place 10 cm3 de toluène et des quantités variables d'acétoxy-1 butadiène-1,3 (isomère trans/isomère cis : 1,55) renfermant 4,6 % en poids de tertiobutyl-4 pyrocatéchol). Les autoclaves étant fermés, on introduit, dans chaque autoclave, 40 bar d'éthylène et 30 bar d'argon.

Ces autoclaves sont placés chacun dans un four puis chauffés à 180°C pendant 6 heures. L'agitation est assurée par balancement.

Après refroidissement, chaque autoclave est dégazé puis chaque masse réactionnelle est analysée par chromatographie en phase vapeur. Les résultats sont donnés dans le tableau III ci-après.

TABLEAU III

----------

| Ex n° | Acétoxy-1 Butadiène-1,3 | Taux de conversion | | S(AC) |
|---|---|---|---|---|
| | | Cis | Trans | |
| 18 | 0,207 mMol | 4,5 % | 46,5 % | 74 % |
| 19 | 1,03 mMol | 6 % | 48,5 % | 72,5 % |
| 20 | 5,21 mMol | 24,5 % | 58,5 % | 36 % |

EXEMPLES 21 - 22 :

On dispose de 2 autoclaves de 25 cm3 en acier inoxydable, revêtu intérieurement de téflon®.

Dans chaque autoclave on place 10 cm3 de toluène, de l'acétoxy-1 butadiène-1,4 (1,04 mMol ; isomère trans/isomère cis : 1,55) renfermant 4,6 % en poids de t-butyl-4 pyrocatéchol . Les autoclaves étant fermés, on introduit, dans chaque autoclave, 50 bar d'argon et 10 bar d'éthylène.

Ces autoclaves sont placés chacun dans un four puis chauffés pendant 17 heures aux températures désirées. L'agitation est assurée par balancement.

Après refroidissement, chaque autoclave est dégazé puis chaque masse réactionnelle est analysée par chromatographie en phase vapeur. Les résultats sont donnés dans le tableau IV ci-après :

TABLEAU IV

-------

| Ex n° | Température | Taux de conversion | | S(AC) |
|---|---|---|---|---|
| | | Cis | Trans | |
| 21 | 200°C | 56 % | 87 % | 23,5 % |
| 22 | 230°C | 79,5 % | 99,5 % | 17,5 % |

EXEMPLES 23 - 24 :

On dispose de 2 autoclaves de 25 cm3 en acier inoxydable.

Dans chaque autoclave on place 10 cm3 de toluène et 0,1176 g (1,05 mMol) d'acétoxy-1 butadiène-1,3 (mélange d'isomères trans/cis = 1,55). Dans l'un des deux autoclaves on ajoute, en plus, du t.butyl-4 pyrocatéchol (3 % molaires/acétoxy-1 butadiène).

On ferme les autoclaves et on introduit alors, dans chaque autoclave, 40 bar d'éthylène et 30 bar d'argon.

Ces autoclaves sont placés dans un four puis chauffés pendant 6 heures à 180°C. L'agitation est assurée par balancement.

Après refroidissement, chaque autoclave est dégazé puis chaque masse réactionnelle est analysée par chromatographie en phase vapeur.

Les résultats sont donnés dans le tableau V ci-après :

TABLEAU V

-------

| Ex n° | t-Butyl-4 pyrocatéchol | Taux de conversion | | S(AC) |
|-------|------------------------|--------------------|----------|-------|
|       |                        | Cis                | Trans    |       |
| 23    | OUI                    | 9 %                | 48 %     | 64 %  |
| 24    | NON                    | 17 %               | 49,5 %   | 49,5 %|

## EXEMPLE 25 :

Dans un autoclave de 25 cm3 en acier inoxydable on place 10 cm3 de toluène, 0,1173 g (1,047 mMol) d'acétoxy-1 butadiène-1,3 (rapport trans/cis : 1,55) et du t-butyl-4 pyrocatéchol (3 molaires/acétoxy-1 butadiène). On introduit 40 bar d'éthylène et 30 bar d'argon.

On chauffe ensuite à 180°C pendant 30 heures.

Les résultats obtenus sont les suivants :
- taux de conversion de l'isomère cis : 24 %
- taux de conversion de l'isomère trans : 95 %
- sélectivité en acétoxy-2 cyclohexène : 70,5 %

## EXEMPLE 26 :

Dans un autoclave de 25 cm3 en acier inoxydable on place 10 cm3 de diméthyl-2,3 butène-2, 0,115 g (1,04 mMol) d'acétoxy-1 butadiène-1,3 (rapport trans/cis : 1,55) et du t-butyl-4 pyrocatéchol (4,6 % en poids/acétoxy-1 butadiène). On introduit 65 bar d'éthylène et 55 bar d'argon.

On chauffe ensuite à 180°C pendant 3 heures sous agitation par balancement.

Les résultats obtenus sont les suivants :
- taux de conversion de l'isomère cis : 34 %
- taux de conversion de l'isomère trans : 47,5 %
- sélectivité en acétoxy-2 cyclohexène : 27 %

## Revendications

1. Procédé de préparation d'un carboxylate de cyclohexène-2 yle par cycloaddition d'un carboxylate de butadiène-1,3 yle-1 et de l'éthylène.

2. Procédé selon la revendication 1, caractérisé en ce que la cycloaddition est réalisée sous pression supérieure à la pression atmosphérique et à une température supérieure ou égale à 100°C.

7

3. Procédé selon la revendication 2, caractérisé en ce que la température est inférieure à 250°C.

4. Procédé selon l'une quelconque des revendications précédentes, caractérisé en ce que la pression partielle de l'éthylène mesurée à 25°C est supérieure ou égale à 5 bar (500 KPa).

5. Procédé selon l'une quelconque des revendications précédentes, caractérisé en ce que la pression partielle de l'éthylène mesurée à 25°C est comprise entre 30 et 70 bar (3000 et 7000 KPa).

6. Procédé selon l'une quelconque des revendications précédentes, caractérisé en ce que la cycloaddition est conduite dans un diluant organique.

7. Procédé selon la revendication 6, caractérisé en ce que le diluant est choisi parmi les hydrocarbures aromatiques, le cas échéant, chlorés ; les hydrocarbures saturés, aliphatiques ou cycloaliphatiques ; les esters ; les éthers et les oléfines dont la double liaison est stériquement suffisamment encombrée pour ne pas réagir en tant que diénophile sur le carboxylate de butadiène-1,3 yle-1.

8. Procédé selon la revendication 6 ou 7 caractérisé en ce que la concentration en carboxylate de butadiène-1,3 yle-1 est inférieure ou égale à 0,5 mol/l.

9. Procédé selon l'une quelconque des revendications précédentes caractérisé en ce que la cycloaddition est réalisée en présence d'un gaz inerte.

10. Procédé selon l'une quelconque des revendications précédentes, caractérisé en ce que la cycloaddition est réalisée en présence d'un inhibiteur radicalaire.

11. Procédé selon l'une quelconque des revendications précédentes, caractérisé en ce que le carboxylate de butadiène-1,3 yle-1 est l'acétoxy-1-butadiène-1,3.

12. Procédé selon la revendication 11, caractérisé en ce que l'acétoxy-1 butadiène 1,3 se présente sous forme d'un mélange d'isomères cis/trans enrichi en isomère trans.

13. Procédé selon l'une quelconque des revendications précédentes, caractérisé en ce que la température de réaction est comprise entre 120 et 230°C.

**Patentansprüche**

1. Verfahren zur Herstellung eines Cyclohexen-2-yl-carboxylates durch Cycloaddition eines Butadien-1,3-yl-1-carboxylates und Ethylen.

2. Verfahren nach Anspruch 1, dadurch gekennzeichnet, daß die Cycloaddition bei höherem Druck als Atmosphärendruck und bei einer Temperatur von 100 °C oder mehr durchgeführt wird.

3. Verfahren nach Anspruch 2, dadurch gekennzeichnet, daß die Temperatur unter 250 °C liegt.

4. Verfahren nach einem der vorhergehenden Ansprüche, dadurch gekennzeichnet, daß der Partialdruck von Ethylen, gemessen bei 25 °C, gleich oder höher als 5 bar (500 KPa) ist.

5. Verfahren nach einem der vorhergehenden Ansprüche, dadurch gekennzeichnet, daß der Partialdruck des Ethylens, gemessen bei 25 °C, zwischen 30 und 70 bar (3000 und 5000 KPa) liegt.

6. Verfahren nach einem der vorhergehenden Ansprüche, dadurch gekennzeichnet, daß die Cycloaddition in einem organischem Verdünnungsmittel durchgeführt wird.

7. Verfahren nach Anspruch 6, dadurch gekennzeichnet, daß das Verdünnungsmittel aus aromatischen ggf. chlorierten Kohlenwasserstoffen; gesättigten, aliphatischen oder cycloaliphatischen Kohlenwasserstoffen; den Estern; den Ethern und den Olefinen, deren Doppelbindung ausreichend sterisch gehindert ist, um nicht als Dienophil mit dem Butadien-1,3-yl-1-carboxylat zu reagieren, gewählt wird.

8. Verfahren nach einem der Ansprüche 6 oder 7, dadurch gekennzeichnet, daß die Konzentration von Butadien-1,3-yl-1-carboxylat kleiner oder gleich 0,5 mol/l ist.

9. Verfahren nach einem der vorhergehenden Ansprüche, dadurch gekennzeichnet, daß die Cycloaddition in Gegenwart eines Inertgases durchgeführt wird.

10. Verfahren nach einem der vorhergehenden Ansprüche, dadurch gekennzeichnet, daß die Cycloaddition in Gegenwart eines Radikalinhibitors durchgeführt wird.

11. Verfahren nach einem der vorhergehenden Ansprüche, dadurch gekennzeichnet, daß das Butadien-1,3-yl-1carboxylat Acetoxy-1-butadien-1,3 ist.

12. Verfahren nach Anspruch 11, dadurch gekennzeichnet, daß Acetoxy-1-butadien-1,3 als cis/trans-Isomerengemisch vorliegt, das mit Transisomeren angereichert ist.

13. Verfahren nach einem der vorhergehenden Ansprüche, dadurch gekennzeichnet, daß die Reaktionstemperatur zwischen 120 und 230 °C liegt.


## Claims

1. Process for the preparation of a 2-cyclohexenyl carboxylate by cycloaddition of a 1,3-butadien-1-yl carboxylate and ethylene.

2. Process according to claim 1, characterised in that the cycloaddition is carried out at a pressure greater than atmospheric pressure and at a temperature greater than or equal to 100°C.

3. Process according to claim 2, characterised in that the temperature is less than 250°C.

4. Process according to any one of the preceding claims, characterised in that the ethylene partial pressure measured at 25°C is greater than or equal to 5 bar (500 kPa).

5. Process according to any one of the preceding claims, characterised in that the ethylene partial pressure measured at 25°C is between 30 and 70 bar (3,000 and 7,000 kPa).

6. Process according to any one of the preceding claims, characterised in that the cycloaddition is carried out in an organic diluent.

7. Process according to claim 6, characterised in that the diluent is chosen from the, if appropriate chlorinated, aromatic hydrocarbons; the saturated, aliphatic or cycloaliphatic, hydrocarbons; the esters; the ethers and the olefins in which the double bond is sufficiently sterically hindered so as not to react as a dienophile with the 1,3-butadien-1-yl carboxylate.

8. Process according to claim 6 or 7, characterised in that the concentration of 1,3-butadien-1-yl carboxylate is less than or equal to 0.5 mol/l.

9. Process according to any one of the preceding claims, characterised in that the cycloaddition is carried out in the presence of an inert gas.

10. Process according to any one of the preceding claims, characterised in that the cycloaddition is carried out in the presence of a radical inhibitor.

11. Process according to any one of the preceding claims, characterised in that the 1,3-butadien-1-yl carboxylate is 1-acetoxy-1,3-butadiene.

12. Process according to claim 11, characterised in that 1-acetoxy-1,3-butadiene is provided in the form of a mixture of cis/trans isomers enriched in the trans isomer.

13. Process according to any one of the preceding claims, characterised in that the reaction temperature is between 120 and 230°C.